# EUROPEAN PATENT SPECIFICATION

(11) **EP 1 424 066 B1**
(45) Date of publication and mention of the grant of the patent: **27.02.2008**
(21) Application number: 04002580.1
(22) Date of filing: 25.12.1996
(51) Int. Cl.: A61K 9/16, A61K 47/18

(54) **Dry compositions comprising hydrophobic stabilisers**
Trockene Zusamensetzungen mit hydrophoben Stabilisatoren
Compositions sèches comprenant des stabilisateurs hydrophobes

(30) Priority: 25.12.1995 JP 33671495
(43) Date of publication of application: 02.06.2004
(62) Divisional of application: 96942636.0
(73) Proprietor: OTSUKA PHARMACEUTICAL CO., LTD., Chiyoda-ku Tokyo 101 (JP)
(72) Inventor: Yamashita, Chikamasa, Naruto-shi Tokushima 772 (JP); Sakata, Kazuya, Itano-gun Tokushima 771-0203 (JP); Ishikawa, Shinichi, Tokushima 773-0011 (JP); Kimura, Yuzo, Tokushima-shi Tokushima 770 (JP)
(74) Representative: HOFFMANN EITLE

(56) References cited:
- WO-A-95/31479
- WO-A-96/32149

## Description

### Technical Field

The present invention relates to a dry composition for inhalants.

### Background Art

Heretofore, several publications have disclosed dry compositions comprising at least one of active ingredients selected from the group consisting of pharmacologically active proteins and pharmacologically active polypeptides in combination with a stabilizer therefor, including human serum albumin, saccharides such as sucrose, mannitol or the like and amino acids such as glycine, alanine, phenylalanine, glutamic acid or the like (Japanese Unexamined Patent Publication No. 102519/1980, European Patent Publication No. 80879A, European Patent Publication No. 82481A, Japanese Unexamined Patent Publication No. 181224/1984, European Patent Publication No. 133767A, European Patent Publication No. 401379A WO 95/31479 A and European Patent Publication No. 168008A). Of those relevant prior arts, the techniques disclosed in Japanese Unexamined Patent Publication No. 102519/1980, European Patent Publication No. 82481A, Japanese Unexamined Patent Publication No. 181224/1984 and European Patent Publication No. 168008A are similar to that of the present invention.

Japanese Unexamined Patent Publication No. 102519/1980 discloses the method in which any one of polyethylene-based nonionic surfactant, antibiotic, chelating agent and aromatic amine is added to an aqueous solution containing interferon and subjected to lyophilization so as to stabilize interferon.

European Patent Publication No. 82481A discloses a lyophilized pharmaceutical composition comprising interferon, an amino acid or the derivative thereof selected from glycine, α-alanine and pharmaceutical acceptable salts thereof in an amount sufficient to stabilize interferon, and a buffer compatible therewith.

Japanese Unexamined Patent Publication No. 181224/1984 discloses a pharmaceutical preparation containing interferon obtained by adding an amino acid or an amino acid and human serum albumin to an aqueous solution containing interferon, followed by lyophilization. Useful amino acids specified in this publication are hydrophilic polar amino acids, such as arginine, asparagine, glutamic acid, glutamine, histidine, lysine, serine and threonine. The publication describes that of those amino acids, glutamic acid is particularly preferred.

European Patent Publication No. 168008A discloses a composition comprising human γ interferon obtained by conducting freezing or lyophilization under the conditions where inorganic salts are substantially absent but amino acids are present. This publication describes that useful amino acids are monoamino aliphatic amino acids. However, the amino acid employed in the examples of this publication is glycine only, and no other amino acids than glycine is employed.

The objects of the above patent applications are all to provide lyophilized pharmaceutical preparations stable enough to be used in the form of injections.

However, the dry compositions disclosed in the above publications have the following serious drawbacks. For example, when the dry composition is left in a highly humid environment, the active ingredient contained in the composition loses its effectiveness and the composition does not retain its dry state due to deliquescence, thereby causing a change in appearance. Further, when the dry composition is preserved in a bottle covered with a rubber stopper without strictly controlling the dryness of the rubber stopper, the dry composition deliquesces due to the moisture contained in the rubber stopper and the active ingredient suffers deterioration in its pharmacological activity. Moreover, in the case where the dry composition in the form of particles is produced by conducting spray-drying from a solution containing the above active ingredient and the stabilizer, as well as in the case where the above solution is subjected to lyophilization followed by milling, the size of the individual grains varies greatly and hence it is difficult for the final product to secure uniformity. In particular, since the obtained product necessarily includes granules of a large particle size and the particle size increases in a highly humid environment, it is difficult to administer this product by an intrapulmonary route or an intrapharynx route.

### Disclosure of the Invention

In view of the foregoing, the inventors conducted extensive research to develop a dry composition free from the drawbacks described above. Consequently, the inventors found that an advantageous dry composition in which the above drawbacks are overcome can be obtained by employing the following specific substances as the stabilizer for the active ingredient in the dry composition. The present invention is accomplished based on the finding.

The present invention relates to a dry composition for inhalants comprising at least one of active ingredients selected from the group consisting of pharmacologically active proteins and pharmacologically active polypeptides and as a stabilizer at least one of hydrophobic stabilizers selected from the group consisting of valine and isoleucine.

In accordance with the present invention, there is provided a dry composition free from the conventional drawbacks described above. For example, even when the dry composition is left in a highly humid environment, the active ingredient contained in the dry composition scarcely loses its pharmacological activity and the dry composition does not deliquesce and retains its dry state over a long period of time. Further, in the case where the dry composition in the form of particles is produced from a solution containing the above active ingredient and the stabilizer by performing spray-drying, and in the case where the solution containing the above active ingredient and the stabilizer is subjected to lyophilization followed by milling, desired particles can be obtained whose particle size distribution is sharp enough to be suitably administered by an intrapulmonary route or an intrapharynx route. Moreover, the stabilizers employed in the present invention are inexpensive, readily available and industrially advantageous.

The dry compositions according to the present invention encompass the following compositions:
(1) A dry composition for inhalants comprising at least one of active ingredients selected from the group consisting of pharmacologically active proteins and pharmacologically active polypeptides and as a stabilizer at least one of hydrophobic stabilizers selected the group consisting of valine and isoleucine.
(2) A dry composition as defined in Item (1) in which the stabilizer is valine.
(3) A dry composition as defined in Item (1) in which the stabilizer is isoleucine.
(4) A dry composition as defined in Item (1) in which the active ingredient is interferon.
(5) A dry composition as defined in Item (1) in which the active ingredient is interleukin.
(6) A dry composition as defined in Items (1) to (5) in which the particle size is in the range of from 0.5 µm to 10 µm.
(7) A dry composition as defined in Items (1) to (5) which is obtained by spray-drying method.
(8) A dry composition as defined in Items (1) to (5) which is obtained by spray-drying method and has the particle size in the range of from 0.5 µm to 10 µm.

For use as at least one of active ingredients in the present invention selected from the group consisting of pharmacologically active proteins and pharmacologically active polypeptides, suitable examples of such active ingredients include proteins such as enzyme, hemoglobin, immunoglobulin, hormone, coagulation factor and polypeptides including antiviral polypeptides such as interferons-α, -β, -γ, immunoregulatory polypeptides such as interleukins 1, 2, 3, 4, 5, 6, 7, 8 and hematopoietic polypeptides. In the present invention, these active ingredients may be used alone or in combination thereof. A variety of peptides can be used in the present invention, which encompass naturally occurring polypeptides, recombinant polypeptides and chemically synthesized polypeptides.

In the dry composition of the present invention, at least one of hydrophobic stabilizers selected the group consisting of valine and isoleucine is included as the stabilizer. In the present invention, it is important to use a hydrophobic stabilizer having a Hydropathy Index ("A Simple Method for Displaying the Hydrophathic Character of a Protein", Jack Kyte and Russel F. Doolittel, J. Mol. Biol., (1982) 157, 105-132) of at least about 3.

In the present invention, the hydrophobic amino acids valine and isoleucine may be used alone or in combination thereof.

The hydrophobic stabilizer is included in the dry composition of the present invention generally in an amount of, but not specifically limited to, from 40 wt% (inclusive) to 100 wt% (exclusive), in some case from 50 wt% (inclusive) to 100 wt% (exclusive), in some case from 60 wt% (inclusive) to 100 wt% (exclusive), and in some case from 70 wt% (inclusive) to 100 wt% (exclusive). Depending on the kind of the active ingredient used, the amount of the hydrophobic stabilizer present in the dry composition of the present invention is, in some case, from 80 wt% (inclusive) to 100 wt% (exclusive).

The amount of the active ingredient contained in the dry composition of the present invention may vary depending on the kind of the active ingredient used and is not generally mentioned. Preferably, the active ingredient is present in the dry composition in an amount of 50 wt% or less, in some case 15 wt% or less, in some case 10 wt% or less, and in some case 5 wt% or less. Even if the same kind of the active ingredient is used, the amount included in the composition may vary, depending on the disease to be treated or the formulations, and a clinically adequate amount of the active ingredient may suitably be included in the dry composition of the present invention. For example, when interferon or interleukin is employed, the suitable amount thereof in the dry composition is 1 to 10x10⁷ IU/mg, in some case 10 to 8x10⁷ IU/mg, in some case 100 to 6x10⁷ IU/mg, in some case 100 to 4x10⁷ IU/mg, in some case 100 to 3x10⁷ IU/mg, in some case 100 to 2x10⁷ IU/mg, and in some case 100 to 1x10⁷ IU/mg.

In the present invention, in order to stabilize the composition before drying, to stabilize the particulate product after drying, or to prevent absorption to containers, there may suitably be added, before or after drying, known stabilizers including human serum albumin, saccharides such as sucrose, mannitol, trehalose, maltose or the like, amino acids (excluding hydrophobic amino acids) such as glycine, alanine, sodium glutamate, gelatine, and surfactants such as polyoxyethylene sorbitan fatty acid esters, sorbitan trioleate, oleyl alcohol, lecithin or the like.

When human serum albumin is used, the amount added is generally in the range of from 0 wt% to 20 wt%, in some case from 0 wt% to 30 wt%, in some case from 0 wt% to 40 wt%, in some case from 0 wt% to 50 wt%, and in some case from 0 wt% to 60 wt%.

When human serum albumin is not used, it is preferred to add at least one of known stabilizers such as saccharides, e.g., sucrose, mannitol, trehalose, maltose, etc., amino acids (excluding hydrophobic amino acids), e.g., glycine, alanine, sodium glutamate, gelatine, and surfactants, e.g., polyoxyethylene sorbitan fatty acid esters, sorbitan trioleate, oleyl alcohol, lecithin, etc. Preferably, the saccharides, amino acids and surfactants described above are employed in combination.

Since the dry composition of the present invention is formulated into inhalants, the dry composition is subjected to the following procedure.

When employing lyophilization method, a raw material in the form of a solution comprising at least one of active ingredients selected from the group consisting of pharmacologically active proteins and pharmacologically active polypeptides in combination with the hydrophobic stabilizer is subjected to lyophilization and the resultant lyophilized product is micronized using a jet-milling equipment, ball-milling equipment or the like.

When employing spray-drying method, a raw material in the form of a solution comprising at least one of active ingredients selected from the group consisting of pharmacologically active proteins and pharmacologically active polypeptides in combination with the hydrophobic stabilizer is spray-dried to produce particles.

Preferred methods for producing the dry composition of the present invention are illustrated below.

The active ingredient and the hydrophobic stabilizer described above are dissolved in water or a mixture of water and lower alcohol. Water can be used singly, but it is preferred to use a mixture of water and lower alcohol in the present invention. Preferred examples of lower alcohols employed in the present invention are alcohols compatible with water, such as, methanol, ethanol, 1-propanol, 2-propanol, butanol, tertiary butanol, etc. The lower alcohol is used alone, but two or more kinds thereof may be used in combination. Of the lower alcohols listed above, ethanol is particularly preferred.

The suitable mixing ratios of water and lower alcohol employed in the present invention are indicated as follows. The weight ratio of the former to the latter is 40 to 95 : 60 to 5, preferably 40 to 80 : 60 to 20, more preferably 60 to 80 : 40 to 20, and most preferably 60 to 70 : 40 to 30. When the mixing proportion of lower alcohol is less than the above range, it is difficult to efficiently produce dry particles having a particle size of 5.0 µm or less. By contrast, when the mixing proportion of lower alcohol is greater than the above range, it is difficult to dissolve the active ingredient in the above-described mixture and turbidity occurs, and consequently, the pharmaceutically active protein or the like contained in the raw material loses its activity.

In the subsequent step of the method of the present invention, the raw material in the form of a solution comprising the active ingredient and the hydrophobic stabilizer is sprayed into a hot air-stream and dried. The media of the hot air-stream are those that contain inert gas such as nitrogen or the like. In the present invention, the air is preferably used. The conditions in which the raw material is sprayed into a hot air-stream are not critical, but preferably spraying is carried out under the conditions of: spraying pressure of 0.5 to 10 kg/cm², preferably 1 to 3 kg/cm²; spraying concentration of 1 to 100 g/min, preferably 5 to 20 g/min; and spray nozzle diameter indicated as an orifice diameter of 50 to 2000 µm, preferably 200 to 100.0 µm.

In the present invention, the temperature at which spray-drying is efficiently conducted is normally in the range between about 100°C and about 300°C, preferably between about 120°C and about 180 °C. The moisture content of the particles after spray-drying is 5 % or less, preferably 2 % or less.

In the present invention, a surfactant may be added, before or after spray-drying, to the composition so that dispersability of the resultant particles is improved. A variety of known surfactants can be used, such as, polyoxyethylene sorbitan fatty acid ester, sorbitan trioleate, oleyl alcohol, lecithin or the like.

According to the method of the present invention described above, the dry composition can readily be micronized.

As the dry composition of the present invention is formulated into an inhalant, the particle size of the final granular product is preferably in the range of from 0.1 µm to 10 µm, more preferably in the range of from 0.5 µm to 10 µm.

### Brief Description of Drawings

Figure 1 is a graph showing the particle size distribution of the dry composition in the form of particles produced by using isoleucine as the amino acid.
Figure 2 is a graph showing the particle size distribution of the dry composition in the form of particles produced by using alanine as the amino acid.
Figure 3 is a graph showing the particle size distribution of the dry composition in the form of particles produced by using proline as the amino acid.

### Best Mode for Carrying Out the Invention

The present invention is further described by reference to the following examples.

### Example 1

A suitable amount of distilled water for injection was poured into respective vials to give 1 ml of an injection comprising 0.1 ml of a drug substance in solution containing interferon-α (hereinafter referred to as "IFN-α bulk solution", titer: 2x10⁷ IU/ml), 5 mg of various amino acids and 1 mg of human serum albumin (HSA) per vial and subjected to lyophilization. Those samples were left to stand for three days under the conditions where the temperature was 40°C, relative humidity (RH) was 75% and the vials were left open (uncapped). Three days after, the titer of IFN-α was determined and the residual activity of INF-α was calculated by setting the IFN-α activity measured after drying to equal 100%. Further, the same samples were evaluated for change in appearance after three days of standing under the conditions where the temperature was 40°C, RH was 75% and the vials were open. The results are shown in Table 1 below.

**Table 1**

| | Hydropathy Index | Initial IFN-α Activity (%) | Residual IFN-α Activity at 40°C, RH 75%, 3 days after(%) | Change in Appearance |
|---|---|---|---|---|
| Isoleucine | 4.5 | 100 | 84.3 | No Change |
| Valine | 4.2 | 100 | 79.5 | No Change |
| Leucine | 3.8 | 100 | 77.6 | No Change |
| Phenylalanine | 2.8 | 100 | 61.9 | No Change |
| Alanine | 1.9 | 100 | 34.9 | Slightly Deliquesced |
| Glycine | -0.4 | 100 | 69.2 | Almost Deliquesced |
| Proline | -1.6 | 100 | 51.3 | Completely Deliquesced |
| Arginine | -4.5 | 100 | 48.8 | Completely Deliquesced |

As is evident from the results summarized in Table 1, the products obtained by the present invention employing the hydrophobic amino acids valine or isoleucine are remarkably superior in stability of IFN-α and/or change in appearance to the products in which other amino acids were employed, even when left in an excessively humid environment.

### Example 2

### (1) Spray-dried products containing IFN-α and isoleucine

Deionized water was added to a mixture of 50 ml of an IFN-α bulk solution (titer: 2x10⁷ IU/ml), 3500 mg of isoleucine and 700 mg of HSA, and then stirred thoroughly, to prepare 700 g of an IFN-α solution. To 700 g of this IFN-α solution was added 300 g of ethanol to give a weight ratio of water to ethanol of 7 : 3, and the solution to be spray-dried was produced.

Using a spray drier (Yamato Pulvis Basic Unit Model GB-21, manufactured by Yamato Science Co., Ltd.) under the conditions of air-supplying temperature of 130°C, spraying pressure of 2 kg/cm² and spraying rate of 10 g/min, the above solution was spray-dried to produce dry particles.

### (2) Spray-dried product containing isoleucine but not containing IFN-α for use as a placebo

Dry particles were produced in the similar manner as in (1) above with the exception that IFN-α was not employed.

The dry particles produced by the processes (1) and (2) above were each evaluated for aerodynamic average particle size (volume basis distribution), and the results are shown in Table 2 below. Aerodynamic average particle size was determined by dispersing the particles using an aerodisperser (Amherst Process Instruments, Inc.) and the measurement was conducted by using an aerosizer (Amherst Process Instruments, Inc.). Measuring conditions are as follows: air-stream shearing force: medium; sample particles supplying rate: medium; deagglomeration: normal; and vibration of dispersing pin: on.

**Table 2**

| | Aerodynamic Average Particle Size (µm) |
|---|---|
| Isoleucine (placebo) | 0.9697 |
| Isoleucine (IFN) | 0.9549 |

Table 2 demonstrates that IFN-α does not affect the aerodynamic average particle size of the spray-dried products and the particle size distribution of the particles is dependent on the nature of amino acids employed.

### Test Example 1

To make a solution containing 0.5 wt% of each amino acid indicated in Table 3 and 0.1 wt% of HSA, suitable amount of deionized water was added to the solution and thoroughly stirred to prepare 700 g of an amino acid solution. To 700 g of this solution was added ethanol to give a weight ratio of water to ethanol of 7 : 3, and the solution to be spray-dried was produced.

Using a spray drier (Yamato Pulvis Basic Unit Model GB-21, manufactured by Yamato Science Co., Ltd.) under the conditions of air-supplying temperature of 130°C, spraying pressure of 2 kg/cm² and spraying rate of 10 g/min, the above solution was spray-dried to produce the dry particles.

The dry particles produced by the above processes were each evaluated for moisture content (moisture content immediately after production and moisture content 24 hours after standing under the condition of RH 96%) and the average particle size distribution (volume basis distribution), and the results are summarized in Table 3 below.

Measurement of moisture content: the water contained in the dry particles were vaporized using Hiranuma auto moisture vaporizing instrument (LE-24S) and the moisture content was measured by using Hiranuma moisture microanalyzer (AQ-6).

Measurement of particle size: by using a laser diffraction scattering particle size distribution measuring equipment (LEM-24S, manufactured by Seishin Co., Ltd.), the particle size distribution of the dry particles (volume basis distribution) was determined. Measuring conditions were as follows: dispersing nozzle pressure: 5.0 kg/cm²; refractive index: 1.33.

**Table 3**

| | Hydropathy Index | Initial Moisture Content (%) | Moisture Content at RH96% 24hrs after(%) | Particle Size Distribution (µm) | | |
|---|---|---|---|---|---|---|
| | | | | x 10 | x 50 | x 90 |
| Isoleucine | 4.5 | 1.38 | 13.64 | 1.2 | 2.0 | 3.1 |
| Valine | 4.2 | 1.90 | 10.18 | 1.2 | 1.8 | 3.1 |
| Leucine | 3.8 | 1.69 | 12.05 | 1.1 | 1.7 | 3.3 |
| Phenylalanine | 2.8 | 2.34 | 13.74 | 1.5 | 2.7 | 7.4 |
| Alanine | 1.9 | 3.11 | 46.27 | 1.2 | 2.0 | 12.2 |
| Glycine | -0.4 | 2.29 | 66.73 | 1.5 | 3.8 | 9.2 |
| Proline | -1.6 | 2.25 | 217.80 | 2.7 | 13.4 | 34.9 |
| Arginine | -4.5 | Spray-dried products cannot be produced. | | | | |

The values shown in Table 3 are cumulative % under sieving. For example, "x 50" indicates a particle size in which the particles of smaller sizes are accumulated to occupy 50% of the volume

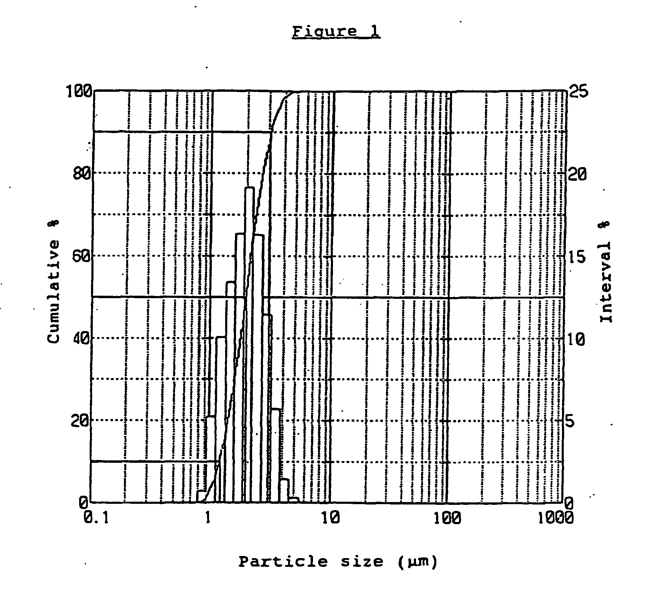

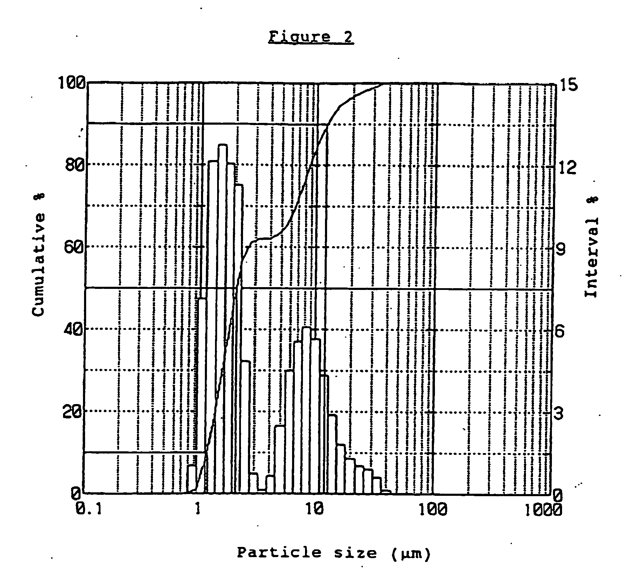

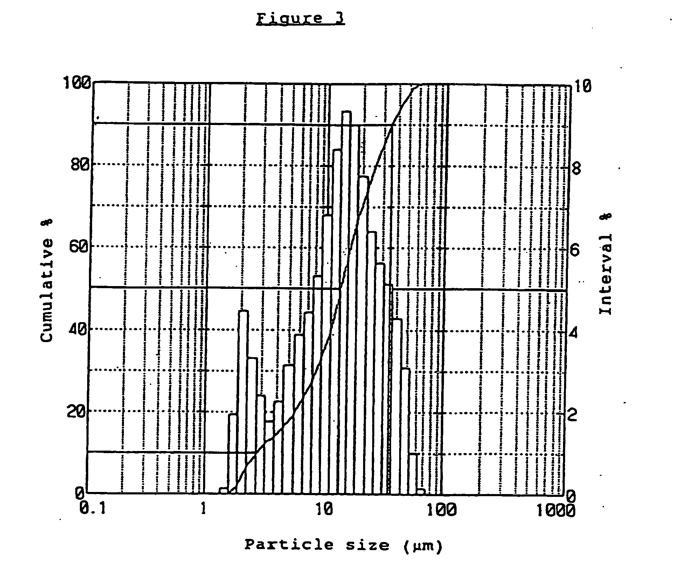

The dry particles produced using isoleucine, alanine or proline as the amino acid were evaluated for the particle size distribution by employing the above procedure and the graphs showing individual particle size distribution are represented in Figures 1, 2 and 3, respectively.

As is evident from the results shown in Table 1 and Figures 1, 2 and 3, the spray-dried products produced by using valine or isoleucine are superior to the products obtained by using other amino acids, in moisture absorption even when the products were left in a highly humid environment and/or in uniformity of the particle size distribution.

### Example 3

Dry particles were produced in the similar manner as in Example 2 with the exception that 300 g of ethanol was not added.

### Comparative Examples 4 to 7

Dry particles were produced in the similar manner as in Example 2 with the exception that leucine, valine, leucyl-valine or isoleucyl-valyl-leucine was used in lieu of isoleucine.

### Examples 8 to 22

Dry particles were produced in the similar manner as in Example 2 with the exception that an IFN-α bulk solution, isoleucine and HSA were employed in the amounts indicated in Table 4.

**Table 4**

| Example | IFN-α (IU) | Isoleucine(mg) | HSA(mg) |
|---|---|---|---|
| 8 | 100 x 10⁷ | 3500 | 0 |
| 9 | 100 x 10⁷ | 3500 | 7 |
| 10 | 100 x 10⁷ | 3500 | 70 |
| 11 | 1 x 10⁷ | 3500 | 700 |
| 12 | 1 x 10⁷ | 3500 | 0 |
| 13 | 1 x 10⁷ | 3500 | 7 |
| 14 | 1 x 10⁷ | 3500 | 70 |
| 15 | 10 x 10⁷ | 3500 | 700 |
| 16 | 10 x 10⁷ | 3500 | 0 |
| 17 | 10 x 10⁷ | 3500 | 7 |
| 18 | 10 x 10⁷ | 3500 | 70 |
| 19 | 1000 x 10⁷ | 3500 | 700 |
| 20 | 1000 x 10⁷ | 3500 | 0 |
| 21 | 1000 x 10⁷ | 3500 | 7 |
| 22 | 1000 x 10⁷ | 3500 | 70 |

### Comparative Examples 23 to 37

Dry particles were produced in the similar manner as in Example 4 with the exception that an IFN-α bulk solution, leucine and HSA were employed in the amounts indicated in Table 5.

**Table 5**

| Comparative Example | IFN-α (IU) | Leucine(mg) | HSA(mg) |
|---|---|---|---|
| 23 | 100 x 10⁷ | 3500 | 0 |
| 24 | 100 x 10⁷ | 3500 | 7 |
| 25 | 100 x 10⁷ | 3500 | 70 |
| 26 | 1 x 10⁷ | 3500 | 700 |
| 27 | 1 x 10⁷ | 3500 | 0 |
| 28 | 1 x 10⁷ | 3500 | 7 |
| 29 | 1 x 10⁷ | 3500 | 70 |
| 30 | 10 x 10⁷ | 3500 | 700 |
| 31 | 10 x 10⁷ | 3500 | 0 |
| 32 | 10 x 10⁷ | 3500 | 7 |
| 33 | 10 x 10⁷ | 3500 | 70 |
| 34 | 1000 x 10⁷ | 3500 | 700 |
| 35 | 1000 x 10⁷ | 3500 | 0 |
| 36 | 1000 x 10⁷ | 3500 | 7 |
| 37 | 1000 x 10⁷ | 3500 | 70 |

### Example 38

A suitable amount of deionized water was added to a mixture of 50 ml of an IFN-α bulk solution (titer: 2x10⁷ IU/ml), 3500 mg of isoleucine and 700 mg of HSA, and stirred thoroughly, to prepare 700 ml of an IFN-α solution. This solution was lyophilized, and the resultant lyophilized product was collected and milled using a jet-milling equipment to obtain dry particles.

### Examples 39 to 53

Dry particles were produced in the similar manner as in Example 38 with the exception that an IFN-α bulk solution, isoleucine and HSA were employed in the amounts indicated in Table 6.

**Table 6**

| Example | IFN-α (IU) | Isoleucine(mg) | HSA(mg) |
|---|---|---|---|
| 39 | 100 x 10⁷ | 3500 | 0 |
| 40 | 100 x 10⁷ | 3500 | 7 |
| 41 | 100 x 10⁷ | 3500 | 70 |
| 42 | 1 x 10⁷ | 3500 | 700 |
| 43 | 1 x 10⁷ | 3500 | 0 |
| 44 | 1 x 10⁷ | 3500 | 7 |
| 45 | 1 x 10⁷ | 3500 | 70 |
| 46 | 10 x 10⁷ | 3500 | 700 |
| 47 | 10 x 10⁷ | 3500 | 0 |
| 48 | 10 x 10⁷ | 3500 | 7 |
| 49 | 10 x 10⁷ | 3500 | 70 |
| 50 | 1000 x 10⁷ | 3500 | 700 |
| 51 | 1000 x 10⁷ | 3500 | 0 |
| 52 | 1000 x 10⁷ | 3500 | 7 |
| 53 | 1000 x 10⁷ | 3500 | 70 |

### Comparative Example 54

Dry particles were produced in the similar manner as in Example 38 by performing lyophilization with the exception that in lieu of isoleucine, 3500 mg of leucine was used.

### Comparative Examples 55 to 69

Dry particles were produced in the similar manner as in Example 54 with the exception that an IFN-α bulk solution, leucine and HSA were employed in the amounts indicated in Table 7.

**Table 7**

| Comparative Example | IFN-α (IU) | Leucine(mg) | HSA(mg) |
|---|---|---|---|
| 55 | 100 x 10⁷ | 3500 | 0 |
| 56 | 100 x 10⁷ | 3500 | 7 |
| 57 | 100 x 10⁷ | 3500 | 70 |
| 58 | 1 x 10⁷ | 3500 | 700 |
| 59 | 1 x 10⁷ | 3500 | 0 |
| 60 | 1 x 10⁷ | 3500 | 7 |
| 61 | 1 x 10⁷ | 3500 | 70 |
| 62 | 10 x 10⁷ | 3500 | 700 |
| 63 | 10 x 10⁷ | 3500 | 0 |
| 64 | 10 x 10⁷ | 3500 | 7 |
| 65 | 10 x 10⁷ | 3500 | 70 |
| 66 | 1000 x 10⁷ | 3500 | 700 |
| 67 | 1000 x 10⁷ | 3500 | 0 |
| 68 | 1000 x 10⁷ | 3500 | 7 |
| 69 | 1000 x 10⁷ | 3500 | 70 |

### Example 70

Dry particles were produced in the similar manner as in Example 2 with the exception that in lieu of the IFN-α bulk solution, 50 ml of an IFN-γ bulk solution (titer: 2 x10⁷ IU/ml) was used.

### Example 71

Dry particles were produced in the similar manner as in Example 2 with the exception that in lieu of the IFN-α bulk solution, 50 ml of a bulk solution containing interleukin-1β in which cysteine at position 71 was substituted with serine (described in European Patent Publication No. 237073A; titer: 1.2 x 10⁸ IU/ml) was used.

### Example 72

Dry particles were produced in the similar manner as in Example 2 with the exception that in lieu of the IFN-α bulk solution, 50 ml of a bulk solution containing interleukin-1α in which asparagine at position 36 was substituted with aspartic acid and cysteine at position 141 was substituted with serine (described in European Patent Publication No. 237073A; titer: 1.3 x 10⁸ IU/ml) was used.

### Example 73

Dry particles were produced in the similar manner as in Example 38 with the exception that in lieu of the IFN-α bulk solution, 50 ml of an IFN-γ bulk solution (titer: 2 x 10⁷ IU/ml) was used.

### Example 74

Dry particles were produced in the similar manner as in Example 38 with the exception that in lieu of the IFN-α bulk solution, 50 ml of a bulk solution containing interleukin-1β in which cysteine at position 71 was substituted with serine (described in European Patent Publication No. 237073A; titer: 1.2 x 10⁸ IU/ml) was used.

### Example 75

Dry particles were produced in the similar manner as in Example 38 with the exception that in lieu of the IFN-α bulk solution, 50 ml of a bulk solution containing interleukin-1β in which asparagine at position 36 was substituted with aspartic acid and cysteine at position 141 was substituted with serine (described in European Patent Publication No. 237073A; titer: 1.2 x 10⁸ IU/ml) was used.

### Examples 76 to 91

Dry particles were produced in the similar manner as in Example 2 with the exception that the IFN-α bulk solution, hydrophobic stabilizers (leucine and valine) and other stabilizers (glycine, sucrose or mannitol) were employed in the amounts indicated in Table 8.

**Table 8**

| Example | IFN-α(IU) | Hydrophobic Stabilizer | | Other Stabilizer | | |
|---|---|---|---|---|---|---|
| | | Leucine(mg) | Valine(mg) | Glycine(mg) | Sucrose(mg) | Mannitol(mg) |
| 76 | 1 x 10⁷ | 3000 | 500 | | | |
| 77 | 10 x 10⁷ | 3000 | 500 | | | |
| 78 | 100 x 10⁷ | 3000 | 500 | | | |
| 79 | 1000 x 10⁷ | 3000 | 500 | | | |
| 80 | 1 x 10⁷ | 2500 | 500 | 500 | | |
| 81 | 10 x 10⁷ | 2500 | 500 | 500 | | |
| 82 | 100 x 10⁷ | 2500 | 500 | 500 | | |
| 83 | 1000 x 10⁷ | 2500 | 500 | 500 | | |
| 84 | 1 x 10⁷ | 2500 | 500 | | 500 | |
| 85 | 10 x 10⁷ | 2500 | 500 | | 500 | |
| 86 | 100 x 10⁷ | 2500 | 500 | | 500 | |
| 87 | 1000 x 10⁷ | 2500 | 500 | | 500 | |
| 88 | 1 x 10⁷ | 2500 | 500 | | | 500 |
| 89 | 10 x 10⁷ | 2500 | 500 | | | 500 |
| 90 | 100 x 10⁷ | 2500 | 500 | | | 500 |
| 91 | 1000 x 10⁷ | 2500 | 500 | | | 500 |

### Examples 92 to 107

Dry particles were produced in the similar manner as in Example 38 with the exception that the IFN-α bulk solution, hydrophobic stabilizers (leucine and valine) and other stabilizers (glycine, sucrose or mannitol) were employed in the amounts indicated in Table 9.

**Table 9**

| Example | IFN-α(IU) | Hydrophobic Stabilizer | | Other Stabilizer | | |
|---|---|---|---|---|---|---|
| | | Leucine(mg) | Valine(mg) | Glycine(mg) | Sucrose(mg) | Mannitol(mg) |
| 92 | 1 x 10⁷ | 3000 | 500 | | | |
| 93 | 10 x 10⁷ | 3000 | 500 | | | |
| 94 | 100 x 10⁷ | 3000 | 500 | | | |
| 95 | 1000 x 10⁷ | 3000 | 500 | | | |
| 96 | 1 x 10⁷ | 2500 | 500 | 500 | | |
| 97 | 10 x 10⁷ | 2500 | 500 | 500 | | |
| 98 | 100 x 10⁷ | 2500 | 500 | 500 | | |
| 99 | 1000 x 10⁷ | 2500 | 500 | 500 | | |
| 100 | 1 x 10⁷ | 2500 | 500 | | 500 | |
| 101 | 10 x 10⁷ | 2500 | 500 | | 500 | |
| 102 | 100 x 10⁷ | 2500 | 500 | | 500 | |
| 103 | 1000 x 10⁷ | 2500 | 500 | | 500 | |
| 104 | 1 x 10⁷ | 2500 | 500 | | | 500 |
| 105 | 10 x 10⁷ | 2500 | 500 | | | 500 |
| 106 | 100 x 10⁷ | 2500 | 500 | | | 500 |
| 107 | 1000 x 10⁷ | 2500 | 500 | | | 500 |

## Claims

1. A dry composition for inhalants comprising at least one active ingredient selected from the group consisting of enzymes, immunoglobulins, hormones, antiviral polypeptides, immunoregulatory polypeptides and hematopoietic polypeptides and at least one hydrophobic stabilizer selected from the group consisting of valine and isoleucine, wherein the dry composition is in the form of particles having a particle size in the range of from 0.1 µm to 10 µm.

2. The dry composition according to claim 1, wherein the active ingredient is interferon.

3. The dry composition according to claim 1, wherein the active ingredient is interleukin.

4. The dry composition according to claim 1, wherein the stabilizer is valine.

5. The dry composition according to claim 1, wherein the stabilizer is isoleucine.

6. The dry composition according to any one of claims 1 to 5, wherein the particle size is in the range of from 0.5 µm to 10 µm.

7. The dry composition according to any one of claims 1 to 5 which is obtained by a spray-drying method.

8. The dry composition according to any one of claims 1 to 5 which is obtained by a spray-drying method and has a particle size in the range of from 0.5 µm to 10 µm.

## Patentansprüche

1. Trockene Zusammensetzung für Inhalationsmittel, umfassend mindestens einen Wirkstoff, der aus der Gruppe ausgewählt ist, die aus Enzymen, Immunoglobulinen, Hormonen, antiviralen Polypeptiden, immunregulatorischen Polypeptiden und hämatopoetischen Polypeptiden besteht, und mindestens ein hydrophobes Stabilisierungsmittel, das aus der Gruppe ausgewählt ist, die aus Valin und Isoleucin besteht, worin die trockene Zusammensetzung in Form von Partikeln mit einer Partikelgröße im Bereich von 0,1 µm bis 10 µm vorliegt.

2. Trockene Zusammensetzung gemäß Anspruch 1, worin der Wirkstoff Interferon ist.

3. Trockene Zusammensetzung gemäß Anspruch 1, worin der Wirkstoff Interleukin ist.

4. Trockene Zusammensetzung gemäß Anspruch 1, worin das Stabilisierungsmittel Valin ist.

5. Trockene Zusammensetzung gemäß Anspruch 1, worin das Stabilisierungsmittel Isoleucin ist.

6. Trockene Zusammensetzung gemäß mindestens einem der Ansprüche 1 bis 5, worin die Partikelgröße im Bereich von 0,5 µm bis 10 µm liegt.

7. Trockene Zusammensetzung gemäß mindestens einem der Ansprüche 1 bis 5, die durch ein Sprühtrocknungsverfahren erhalten wird.

8. Trockene Zusammensetzung gemäß mindestens einem der Ansprüche 1 bis 5, die durch ein Sprühtrocknungsverfahren erhalten wird und eine Partikelgröße im Bereich von 0,5 µm bis 10 µm aufweist.

## Revendications

1. Composition sèche destinée à des solutions pour inhalation comprenant au moins un ingrédient actif choisi parmi le groupe consistant en les enzymes, les immunoglobulines, les hormones, les polypeptides antiviraux, les polypeptides immunorégulateurs et les polypeptides hématopoïétiques, et au moins un stabilisant hydrophobe choisi parmi le groupe consistant en la valine et l'isoleucine, dans laquelle la composition sèche est sous la forme de particules ayant une taille de particules située dans la plage allant de 0,1 µm à 10 µm.

2. Composition sèche selon la revendication 1, dans laquelle l'ingrédient actif est un interféron.

3. Composition sèche selon la revendication 1, dans laquelle l'ingrédient actif est une interleukine.

4. Composition sèche selon la revendication 1, dans laquelle le stabilisant est de la valine.

5. Composition sèche selon la revendication 1, dans laquelle le stabilisant est de l'isoleucine.

6. Composition sèche selon l'une quelconque des revendications 1 à 5, dans laquelle la taille des particules est située dans la plage allant de 0,5 µm à 10 µm.

7. Composition sèche selon l'une quelconque des revendications 1 à 5, obtenue par un procédé de séchage par pulvérisation.

8. Composition sèche selon l'une quelconque des revendications 1 à 5, obtenue par un procédé de séchage par pulvérisation et dont la taille de particules est située dans la plage allant de 0,5 µm à 10 µm.
